(19)

Europäisches
Patentamt
European
Patent Office
Office européen
des brevets

(11) **EP 4 364 587 A1**

(12) **EUROPEAN PATENT APPLICATION**

(43) Date of publication:
**08.05.2024 Bulletin 2024/19**

(21) Application number: **22205401.7**

(22) Date of filing: **03.11.2022**

(51) International Patent Classification (IPC):
**A24F 40/10** (2020.01) **A24F 40/40** (2020.01)
**A61M 15/02** (2006.01) **B05B 5/00** (2006.01)

(52) Cooperative Patent Classification (CPC):
**A24F 40/40; A24F 40/10; B05B 5/043;
B05B 7/1686;** A61M 11/001; A61M 11/042;
A61M 15/02

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **JT International SA
1202 Geneva (CH)**

(72) Inventors:
• **MCEVOY, Jaakko
  1160 Vienna (AT)**
• **CEGLAR, Tilen
  1150 Vienna (AT)**

(74) Representative: **Serjeants LLP
Dock
75 Exploration Drive
Leicester, LE4 5NU (GB)**

(54) **AEROSOL GENERATING DEVICE**

(57)     An aerosol generating device (1) is described. The device (1) includes an aerosol generator (22) configured to atomise aerosol generating material by heating to generate an aerosol for inhalation by a user. A charging device (26) is configured to apply electrostatic charge to the generated aerosol. A cooling passageway (28) fluidly connects the aerosol generator (22) and the charging device (26). The device (1) includes an outlet (20) through which the charged aerosol is inhaled by the user. A first end (30) of the charging device (26) is positioned a first distance (dl) from the aerosol generator (22) and a second end (32) of the charging device (26) is positioned a second distance (d2) from the end of the outlet (20). The first distance (dl) is greater than the second distance (d2).

FIG. 1

EP 4 364 587 A1

## Description

## Technical Field

The present disclosure relates generally to an aerosol generating device for generating an aerosol for inhalation by a user.

The present disclosure is particularly applicable to a portable (hand-held) aerosol generating device.

## Technical Background

Devices which heat, rather than burn, an aerosol generating material to produce an aerosol for inhalation have become popular with consumers in recent years. A commonly available reduced-risk or modified-risk device is the heated material aerosol generating device, or so-called heat-not-burn device. Devices of this type generate an aerosol or vapour by heating an aerosol generating material to a temperature typically in the range 150°C to 300°C. This temperature range is quite low compared to an ordinary cigarette. Heating the aerosol generating material to a temperature within this range, without burning or combusting the aerosol generating material, generates a vapour which typically cools and condenses to form an aerosol for inhalation by a user of the device. Such devices may use one of a number of different approaches to provide heat to the aerosol generating material, for example a heater.

Five main mechanisms are considered to control particle deposition of an inhaled aerosol in the human respiratory tract: inertial impaction, gravitational sedimentation, diffusion deposition, interception, and electrostatic attraction. It is known that applying an electrostatic charge to aerosol particles or droplets can provide improved deposition in the respiratory tract of the user after they are inhaled. Applying an electrostatic charge may result in repulsion between the aerosol particles or droplets, improving aerosol dispersion, and in the attraction of the inhaled particles or droplets to a surface such as a tissue lining of the respiratory tract. This may lead to an improved user experience regarding aerosol inhalation. An aim of the present disclosure is to provide an aerosol generating device with a charging device configured to apply electrostatic charge to generated aerosol particles or droplets to improve their deposition in the respiratory tract of the user, e.g., in the upper respiratory tract, and to control the deposition location.

## Summary of the Disclosure

According to a first aspect of the present disclosure, there is provided an aerosol generating device comprising:

an aerosol generator configured to atomise aerosol generating material by heating to generate an aerosol for inhalation by a user;

a charging device configured to apply electrostatic charge to the generated aerosol (e.g., to charge the aerosol particles or droplets);

a cooling passageway that fluidly connects the aerosol generator and the charging device; and

an outlet through which the charged aerosol is inhaled by the user;

wherein a first end of the charging device is positioned a first distance from the aerosol generator and a second end of the charging device, opposite the first end, is positioned a second distance from an end of the outlet, the first distance being greater than the second distance.

It will be understood that the aerosol particles or droplets generated by the aerosol generator will have a particle size distribution. Any reference herein to particle diameter is therefore a reference to the mean mass diameter (or MMD), which is the particle diameter for which half the aerosol mass is contained in smaller diameter particles and half is contained in larger diameter particles. The particle size or particle size distribution may be measured or determined using any suitable method known to the skilled person, e.g., laser diffraction or phase doppler anemometry (PDA). Any suitable measurement system may be used to measure or determine particle size. For example, for laser diffraction, where the diffraction pattern differs with particle size, a suitable measurement system might be the Spraytec® laser diffraction system provided by Malvern Instruments Limited of Grovewood Road, Malvern, Worcestershire, WR14 1XZ, United Kingdom. The Spraytec® laser diffraction system comprises:

- a transmitter module which contains a laser light source, e.g., a 4mW He-Ne laser with a wavelength of 632.8 nm,
- a receiver module which can focus any light scattered by the aerosol particles or droplets onto a series of detectors (e.g., 36 element log-spaced arrays) that measure the intensity of light scattered by the aerosol particles or droplets over a wide range of angles, and
- software which controls the system and analyses the scattering data to calculate particle size distributions. The software may use Mie and/or Fraunhofer scattering equations, for example.

The diameter of the aerosol particles or droplets will increase as the generated aerosol moves along the cooling passageway towards the downstream charging device. In particular, the aerosol particles or droplets lose energy as they move along the cooling passageway and will therefore tend to be attracted to each other and condense or coalesce to form larger diameter particles. It has been found that there is an unexpected improvement in deposition of the inhaled aerosol if the electrostatic charge is applied to aerosol particles or droplets that have reached a sufficiently large diameter and have preferably cooled to a temperature at which the user can inhale them. In particular, the first distance between the aerosol generator and the charging device is preferably selected so that the electrostatic charge is applied to aerosol particles or droplets having a diameter greater than about 600 nm. In some cases, the electrostatic charge may be applied to aerosol particles or droplets having a diameter greater than about 700 nm, greater than about 800 nm, or greater than about 1000 nm.

The electrostatic charge may be applied to aerosol particles or droplets having a diameter greater than about 600 nm and less than about 1400 nm.

As already mentioned above, it has been found that applying an electrostatic charge to aerosol particles or droplets having such diameters provides a significant and unexpected improvement in the deposition of the inhaled aerosol in the respiratory tract of the user, and can be used to control the deposition location. The actual numerical value of the first distance may depend on various parameters such as the particular aerosol generator and the aerosol generating material that is being heated, for example. If the aerosol generator uses a heating arrangement or one or more heaters to heat the aerosol generating material, the first distance may depend on the power that is supplied to the heating arrangement or the heater(s).

For any particular aerosol generating device, the range of actual numerical values of the first distance that provide a workable solution (i.e., so that the aerosol generator and the downstream charging device are spaced sufficiently apart that electrostatic charge is applied to aerosol particles or droplets of the desired diameter) may be determined by routine experimentation. The first distance may be greater than a minimum distance where the aerosol particles or droplets have the desired diameter, e.g., 600 nm. In a practical aerosol generating device, the first distance may be less than a maximum distance of about 100 mm to prevent the device from becoming too large for the user to hold comfortably.

The second distance may be as short as possible - i.e., the charging device may be positioned as close as possible to the end of the outlet - in order to mitigate the deposition of charged particles or droplets in the aerosol generating device. In practice, this also means that after being charged by the charging device, the aerosol particles or droplets preferably exit the device through the outlet and are inhaled by the user without any further significant cooling or increase in diameter. Coating the interior of the outlet in a suitable material, e.g., polytetrafluoroethylene (PTFE), may help to prevent the particles or droplets from being deposited in the device. The outlet may be defined by a mouthpiece at the proximal end of the device. In a practical aerosol generating device, the second distance may be less than about 10 mm, and more preferably between about 7 mm and about 3 mm.

The aerosol generating device may further comprise a controller configured to control the electrostatic charge applied to the particles or droplets of the generated aerosol by the charging device. The applied electrostatic charge may be the saturation charge (or "Rayleigh limit") $q_R$ as given by the following equation,

$$q_R = 8\pi\varepsilon^{1/2}\sigma^{1/2}r^{3/2}$$

where $\varepsilon$ is the electric permittivity of the aerosol medium, $\sigma$ is the surface tension, and $r$ is the radius of the aerosol particles.

The applied electrostatic charge may be between $q_R$ and about $q_R/120$. Such an electrostatic charge provides improved deposition of the inhaled aerosol.

The applied electrostatic charge may be between about $q_R/80$ and about $q_R/120$, and more preferably about $q_R/100$. Such an electrostatic charge provides improved deposition of the inhaled aerosol, and also reduces the power consumption of the charging device.

The electrostatic charge applied by the charging device may be a unipolar charge of either polarity (i.e., a positive or negative charge), or a bipolar charge.

The charging device may be an induction charging device, e.g., where the charging device includes an induction ring.

The charging device may be a corona charging device, e.g., where the charging device includes a positive voltage probe and a neutral or negative voltage plate.

The aerosol generating material may be provided as an aerosol generating article (or "consumable"). The aerosol generating device may be adapted to receive, in use, the aerosol generating article. When the aerosol generating article is received in the aerosol generating device, for example in an aerosol generating space or heating chamber of the device, the aerosol generator may be configured to atomise the aerosol generating material by heating. The aerosol generator may comprise a heating arrangement configured to heat the aerosol generating material to generate an aerosol. The heating arrangement may be an induction heating arrangement with an induction coil disposed around or adjacent to the heating chamber, or may comprise one or more heaters. The heating arrangement or the one or more heaters may have a power rating greater than about 5 W, and more preferably between about 8 W and about 12 W.

The aerosol generating material may comprise any type of solid or semi-solid material. Example types of aerosol generating solids include powder, granules, pellets, shreds, strands, particles, gel, strips, loose leaves, cut filler, porous material, foam material or sheets. The aerosol generating material may comprise a plant derived material, and in particular may comprise a tobacco material. It may advantageously comprise reconstituted tobacco, for example including tobacco and any one or more of cellulose fibres, tobacco stalk fibres and inorganic fillers. The aerosol generating material may be a liquid material or substrate.

The aerosol generating material may comprise an aerosol-former. Examples of aerosol-formers include polyhydric alcohols and mixtures thereof such as glycerine or propylene glycol. Typically, the aerosol generating material may comprise an aerosol-former content of between approximately 5% and approximately 50% on a dry weight basis. In some embodiments, the aerosol generating material may comprise an aerosol-former content of between approximately 10% and approximately 20% on a dry weight basis, and possibly approximately 15% on a dry weight basis.

When heated, the aerosol generating material may release one or more volatile compounds. The volatile compounds may include nicotine or flavour compounds such as tobacco or other flavouring.

According to a second aspect of the present disclosure, there is provided an aerosol generating device comprising:

an aerosol generator configured to atomise aerosol generating material by heating to generate an aerosol for inhalation by a user; and
a charging device configured to apply electrostatic charge to the generated aerosol (e.g., to charge the aerosol particles or droplets), an end of the charging device being positioned a distance from the aerosol generator such that the charging device is configured to apply electrostatic charge to aerosol particles of the generated aerosol having a diameter greater than about 600 nm.

The charging device may be configured to apply electrostatic charge to aerosol particles of the generated aerosol having a diameter less than about 1400 nm.

The aerosol generating device may further comprise a cooling passageway that fluidly connects the aerosol generator and the charging device, and an outlet through which the charged aerosol is inhaled by the user. The aerosol generating device may further comprise a controller configured to control the electrostatic charge applied to the particles or droplets of the generated aerosol by the charging device. Other features of the aerosol generating device may be as described herein.

According to a third aspect of the present disclosure, there is provided a method of operating an aerosol generating device comprising:

atomising aerosol generating material by heating to generate an aerosol for inhalation by a user; and
applying electrostatic charge to the generated aerosol;
wherein the electrostatic charge applied to the particles of the generated aerosol is between $q_R$ and about $q_R/120$, wherein $q_R = 8\pi\varepsilon^{1/2}\sigma^{1/2}r^{3/2}$ and $\varepsilon$ is the electric permittivity of the aerosol medium, $\sigma$ is the surface tension, and $r$ is the radius of the aerosol particles.

The electrostatic charge applied to the particles of the generated aerosol is preferably between about $q_R/80$ and about $q_R/120$, and more preferably about $q_R/100$.

The electrostatic charge may be applied to aerosol particles having a diameter greater than about 600 nm, and optionally less than about 1400 nm.

## Brief Description of the Drawings

Figure 1 is a diagrammatic view of an aerosol generating device;
Figure 2 is a graph comparing particle diameter and deposition fraction; and
Figure 3 is a graph comparing particle diameter and the distance between the aerosol generator and the charging device of the aerosol generating device of Figure 1.

## Detailed Description of Embodiments

Embodiments of the present disclosure will now be described by way of example only and with reference to the accompanying drawings.

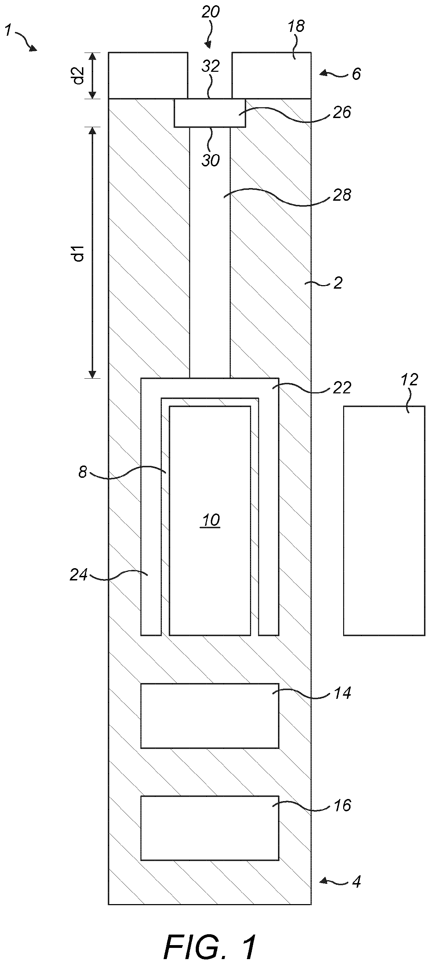

Referring initially to Figure 1, there is shown diagrammatically an example of an aerosol generating device 1. The aerosol generating device 1 comprises a main body 2 housing various components of the aerosol generating device. The main body 2 may have any shape that is sized to fit the components described herein and to be comfortably held by a user unaided, in a single hand.

A first end 4 of the aerosol generating device 1, shown towards the bottom of Figure 1, is described for convenience as a distal, bottom, base or lower end of the aerosol generating device. A second end 6 of the aerosol generating device 1, shown towards the top of Figure 1, is described as a proximal, top or upper end of the aerosol generating device. During use, the user typically orients the aerosol generating device 1 with the first end 4 downward and/or in a distal position with respect to the mouth of the user and the second end 6 upward and/or in a proximate position with respect to the mouth of the user.

The aerosol generating device 1 comprises a heating chamber 8 positioned in the main body 2. The heating chamber 8 defines an interior volume in the form of a cavity 10 for receiving an aerosol generating article 12 that typically comprises a pre-packaged aerosol generating material or substrate. The aerosol generating article 12 is a disposable and replaceable article (also known as a "consumable") which may, for example, contain tobacco as the aerosol generating material 12.

The aerosol generating device 1 further comprises a power source 14, for example one or more batteries which may be rechargeable, and a controller 16. The controller 16 may include a microcontroller unit (MCU) and/or a micro processing unit (MPU). The second end 6 of the aerosol generating device 1 includes a mouthpiece segment 18 which defines an outlet 20 through which a generated aerosol may be inhaled by the user.

The aerosol generating device 1 includes an aerosol generator 22 with a heater 24, which is configured to heat the aerosol generating material when the aerosol generating article 12 is received in the heating chamber 8. Any suitable heater may be used, e.g., a ceramic heater, a wick and coil arrangement or a vertical coil heater.

A charging device 26 is configured to apply electrostatic charge to the aerosol generated by the aerosol generator 22. The charging device 26 may be an induction charging device with an induction ring. A cooling passageway 28 fluidly connects the outlet of the aerosol generator 22 with the charging device 26. Aerosol generated by the aerosol generator 22 passes through the cooling passageway 28 where it cools and the diameter of the particles or droplets increases. At the outlet of the aerosol generator 22 (or at the inlet end of the cooling passageway 28) the aerosol particles or droplets may have a diameter of about 200 nm, for example.

A first end 30 of the charging device 26 is positioned a first distance d1 from the aerosol generator 22. A second end 32 of the charging device is positioned a second distance d2 from the open end of the outlet 20. The first distance d1 corresponds to the length of the cooling passage 20 and is greater than the second distance d2. In other words, the charging device 26 is closer to the open end of the outlet 20 than it is to the aerosol generator 22.

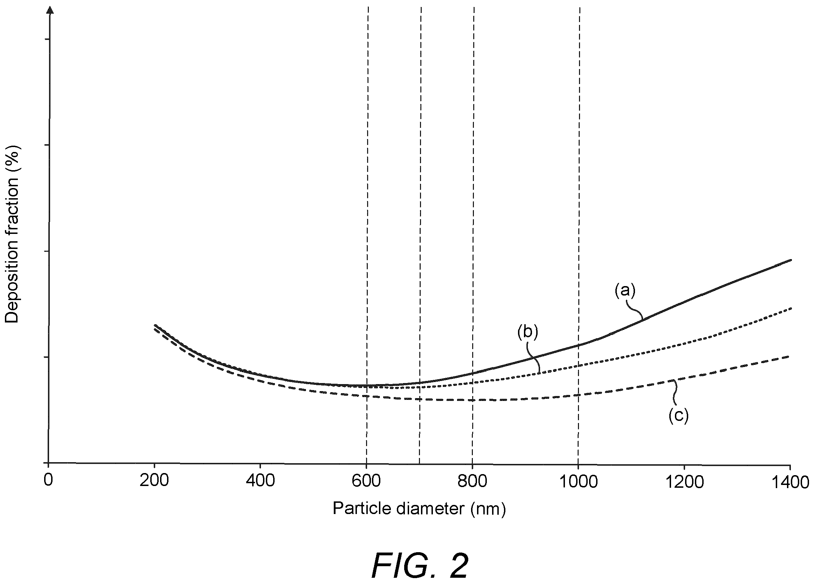

Figure 2 shows the relationship between the diameter of the aerosol particles or droplets and deposition fraction (i.e., the fraction of particles or droplets that locate properly in the upper respiratory tract of the user) for three different applied charges, namely:

(a) a saturation charge where the particles or droplets are charged to the Rayleigh limit $q_R$ (see above),
(b) a preferred charge where the particles or droplets are charged to $q_R/100$, and
(c) a Boltzmann charge where the particles or droplets have a net charge of zero in accordance with the Boltzmann distribution. In other words, the fraction of particles carrying either a negative or positive charge is the same. The particles or droplets therefore behave in the same manner as if they have no charge. It means that the influence of the Boltzmann charge is negligible in terms of improving the deposition of the aerosol in the respiratory tract of the user.

For applied charges (a) and (b), an improved deposition of aerosol particles in the respiratory tract of the user can be seen for particle diameters greater than about 600 nm. The first distance d1 between the aerosol generator 22 and the charging device 26 is therefore preferably selected such that the electrostatic charge is applied to aerosol particles or droplets having a diameter of at least 600 nm. Although it can be seen that there is some improvement in deposition for smaller aerosol particles, e.g., those having a diameter of less than about 400 nm, it is not normally practical to apply an electrostatic charge to aerosol particles or droplets that are too close to the aerosol generator because they are too hot to be inhaled by the user. Typically the aerosol should be cooled to between about 30 to 50°C before exiting the outlet 20.

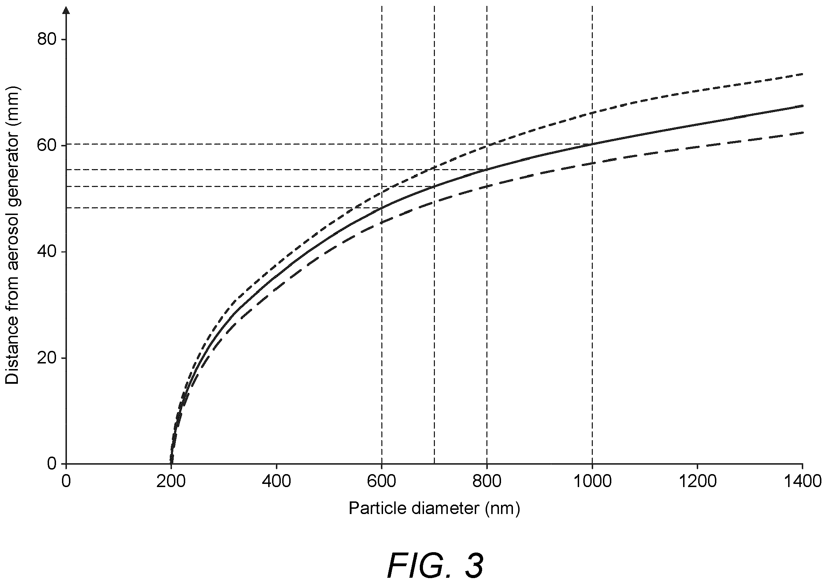

In Figure 3, the solid line indicates the relationship between the diameter of the aerosol particles or droplets and the first distance d1. For a heater 24 with a power rating of 10 W, it can be seen that the first distance d1 should be greater than about 48 mm so that the aerosol generator 22 and the charging device 26 are spaced sufficiently apart that electrostatic charge is applied to aerosol particles or droplets having a diameter greater than about 600 nm. For the aerosol particles or droplets to have a diameter greater than about 700 nm, the first distance d1 should be greater than about 52 mm. For the aerosol particles or droplets to have a diameter greater than about 800 nm, the first distance d1 should be greater than about 56 mm. For the aerosol particles or droplets to have a diameter of greater than about 1000 nm, the first distance d1 should be greater than about 60 mm. For other types of heating arrangement, or heaters with different power ratings, for example, different first distances will be used. In Figure 3 the dashed lines indicate the relationship between the diameter of the aerosol particles or droplets and the first distance d1 for heaters with different power ratings.

The second distance d2 may be as short as possible to mitigate the deposition of charged particles or droplets in the outlet 20 of the aerosol generating device 1. The second distance d2 may be about 5 mm, for example.

Although exemplary embodiments have been described in the preceding paragraphs, it should be understood that various modifications may be made to those embodiments without departing from the scope of the appended claims. Thus, the breadth and scope of the claims should not be limited to the above-described exemplary embodiments.

Any combination of the above-described features in all possible variations thereof is encompassed by the present disclosure unless otherwise indicated herein or otherwise clearly contradicted by context.

Unless the context clearly requires otherwise, throughout the description and the claims, the words "comprise", "comprising", and the like, are to be construed in an inclusive as opposed to an exclusive or exhaustive sense; that is to say, in the sense of "including, but not limited to".

## Claims

1. An aerosol generating device (1) comprising:

   an aerosol generator (22) configured to atomise aerosol generating material by heating to generate an aerosol for inhalation by a user;
   a charging device (26) configured to apply electrostatic charge to the generated aerosol;
   a cooling passageway (28) that fluidly connects the aerosol generator (22) and the charging device (26); and
   an outlet (20) through which the charged aerosol is inhaled by the user;
   wherein a first end (30) of the charging device (26) is positioned a first distance (d1) from the aerosol generator (22) and a second end (32) of the charging device (26), opposite the first end (30), is positioned a second distance (d2) from an end of the outlet (20), the first distance (d1) being greater than the second distance (d2).

2. An aerosol generating device (1) according to claim 1, wherein the first distance (d1) is such that the charging device (26) is configured to apply electrostatic charge to aerosol particles having a mean mass diameter of greater than about 600 nm.

3. An aerosol generating device (1) according to claim 1 or claim 2, wherein the first distance (d1) is such that the charging device (26) is configured to apply electrostatic charge to aerosol particles having a mean mass diameter of greater than about 700 nm.

4. An aerosol generating device (1) according to any preceding claim, wherein the first distance (d1) is such that the charging device (26) is configured to apply electrostatic charge to aerosol particles having a mean mass diameter of greater than about 800 nm.

5. An aerosol generating device (1) according to any preceding claim, wherein the first distance (d1) is such that the charging device (26) is configured to apply electrostatic charge to aerosol particles having a mean mass diameter of greater than about 1000 nm.

6. An aerosol generating device according to any of claims 2 to 5, wherein the first distance (d1) is such that the charging device (26) is configured to apply electrostatic charge to aerosol particles having a mean mass diameter less than about 1400 nm.

7. An aerosol generating device according to any preceding claim, further comprising a controller (16) configured to control the electrostatic charge applied to the particles of the generated aerosol by the charging device (26) to be between about $q_R/80$ and about $q_R/120$, and more preferably about $q_R/100$, wherein $q_R = 8\pi\varepsilon^{1/2}\sigma^{1/2}r^{3/2}$ and $\varepsilon$ is the electric permittivity of the aerosol medium, $\sigma$ is the surface tension, and r is the radius of the aerosol particles.

8. An aerosol generating device (1) according to any preceding claim, wherein the outlet (20) is defined by a mouthpiece (18) at the proximal end (4) of the device (1).

9. An aerosol generating device (1) according to any preceding claim, wherein the charging device (26) is an induction charging device or a corona charging device.

10. An aerosol generating system comprising:

    an aerosol generating article (12) comprising aerosol generating material; and
    an aerosol generating device (1) according to any preceding claim adapted to receive, in use, the aerosol generating article (12).

11. A method of operating an aerosol generating device (1) comprising:

    atomising aerosol generating material by heating to generate an aerosol for inhalation by a user; and
    applying electrostatic charge to the generated aerosol;
    wherein the electrostatic charge applied to the particles of the generated aerosol is between about $q_R$ and about $q_R/120$, wherein $q_R = 8\pi\varepsilon^{1/2}\sigma^{1/2}r^{3/2}$ and $\varepsilon$ is the electric permittivity of the aerosol medium, $\sigma$ is the surface tension, and $r$ is the radius of the aerosol particles.

12. A method according to claim 11, wherein the electrostatic charge applied to the particles of the generated aerosol is between about $q_R/80$ and about $q_R/120$, and more preferably about $q_R/100$.

13. A method according to claim 11 or claim 12, wherein the electrostatic charge is applied to aerosol particles having a mean mass diameter of greater than about 600 nm.

14. A method according to claim 13, wherein the electrostatic charge is applied to aerosol particles having a mean mass diameter less than about 1400 nm.

15. A method according to any of claims 11 to 14, wherein the electrostatic charge is applied by an induction charging device or a corona charging device.

**FIG. 1**

*FIG. 2*

FIG. 3

Europäisches
Patentamt

European
Patent Office

Office européen
des brevets

## EUROPEAN SEARCH REPORT

**Application Number**

EP 22 20 5401

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/209791 A1 (COURBAT CHRISTIAN [CH] ET AL) 11 July 2019 (2019-07-11) * paragraph [0116] - paragraph [0122]; figures 4-6 * | 1-15 | INV. A24F40/10 A24F40/40 |
| A | US 2022/273031 A1 (BIALEK JAKUB [CH] ET AL) 1 September 2022 (2022-09-01) * paragraph [0064] - paragraph [0077]; figures 1,2 * | 1-15 | ADD. A61M15/02 B05B5/00 |
| A | US 5 267 555 A (PAJALICH PHILIP [US]) 7 December 1993 (1993-12-07) * column 3, line 26 - column 4, line 48; figures 1,2 * | 1-15 | |

TECHNICAL FIELDS
SEARCHED        (IPC)

A24F
A61M
B65D
B05B

The present search report has been drawn up for all claims

| Place of search | Date of completion of the search | Examiner |
|---|---|---|
| Munich | 28 March 2023 | Dobbs, Harvey |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 5401

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2023

| Patent document cited in search report | | Publication date | Patent family member(s) | | Publication date |
|---|---|---|---|---|---|
| US 2019209791 | A1 | 11-07-2019 | AR | 113538 A1 | 13-05-2020 |
| | | | BR | 112020007110 A2 | 24-09-2020 |
| | | | CN | 111343876 A | 26-06-2020 |
| | | | EP | 3716799 A1 | 07-10-2020 |
| | | | JP | 7196172 B2 | 26-12-2022 |
| | | | JP | 2021503883 A | 15-02-2021 |
| | | | KR | 20200092936 A | 04-08-2020 |
| | | | RU | 2020117405 A | 30-12-2021 |
| | | | TW | 201924550 A | 01-07-2019 |
| | | | US | 2019209791 A1 | 11-07-2019 |
| | | | WO | 2019105812 A1 | 06-06-2019 |
| US 2022273031 | A1 | 01-09-2022 | CN | 114173586 A | 11-03-2022 |
| | | | EP | 4013247 A1 | 22-06-2022 |
| | | | JP | 2022544744 A | 21-10-2022 |
| | | | KR | 20220044285 A | 07-04-2022 |
| | | | US | 2022273031 A1 | 01-09-2022 |
| | | | WO | 2021028217 A1 | 18-02-2021 |
| US 5267555 | A | 07-12-1993 | NONE | | |

EPO FORM P0459

For more details about this annex : see Official Journal of the European Patent Office, No. 12/82